# EUROPÄISCHE PATENTSCHRIFT

(11) **EP 1 903 336 B1**
(45) Veröffentlichungstag und Bekanntmachung des Hinweises auf die Patenterteilung: **26.10.2011**
(21) Anmeldenummer: 07115163.3
(22) Anmeldetag: 29.08.2007
(51) Int. Cl.: G01N 33/50, G01N 33/533, G01N 33/68

(54) **Verfahren zur räumlich hochauflösenden Untersuchung von einer mit einer fluoreszierenden Substanz markierten Struktur einer Probe**
Method for spatially high-resolution investigation of the structure of a sample marked with a fluorescent substance
Procédé d'examen haute résolution dans l'espace d'une structure marquée par une substance fluorescente d'un échantillon

(30) Priorität: 25.09.2006 DE 102006045607
(43) Veröffentlichungstag der Anmeldung: 26.03.2008
(73) Patentinhaber: Leica Microsystems CMS GmbH, 35578 Wetzlar (DE)
(72) Erfinder: Knebel, Werner, 76709 Kronau (DE); Schröder, Jan, 68199 Mannheim (DE)
(74) Vertreter: Hassenbürger, Anneliese

(56) Entgegenhaltungen:
- WO-A-01/96871
- DE-A1- 10 313 138
- FILHOL O ET AL: "Live-cell fluorescence imaging reveals the dynamics of protein kinase CK2 individual subunits" MOLECULAR AND CELLULAR BIOLOGY, AMERICAN SOCIETY FOR MICROBIOLOGY, WASHINGTON, US, Bd. 23, Nr. 3, Februar 2003 (2003-02), Seiten 975-987, XP002353579 ISSN: 0270-7306
- KEPPLER, A. ET AL: "A general method for the covalent labeling of fusion proteins with small molecules in vivo" NATURE BIOTECHNOLOGY, Bd. 21, März 2003 (2003-03), Seiten 86-89, XP002462235
- KEPPLER, A. ET AL: "Labeling of fusion proteins with synthetic fluorophores in live cells" PNAS, Bd. 101, Nr. 27, 6. Juli 2004 (2004-07-06), Seiten 9955-9959, XP002462236

## Beschreibung

Die Erfindung betrifft ein Verfahren zur räumlich hochauflösenden Untersuchung von einer mit einer fluoreszierenden Substanz markierten Struktur einer Probe, wobei die Substanz wiederholt von einem ersten Zustand in einen zweiten Zustand überführbar ist, wobei sich die ersten und die zweiten Zustände in mindestens einer optischen Eigenschaft voneinander unterscheiden, umfassend die Schritte, dass die Substanz in einem zu erfassenden Probenbereich zunächst in den ersten Zustand gebracht wird und dass mittels eines optischen Signals der zweite Zustand induziert wird, wobei innerhalb des zu erfassenden Probenbereichs räumlich begrenzte Teilbereiche gezielt ausgespart werden.

Des Weiteren betrifft die Erfindung ein Verfahren zur räumlich hochauflösenden Untersuchung von einer mit einer fluoreszierenden Substanz markierten Struktur einer Probe, wobei die Substanz von einem ersten Zustand in einen zweiten fluoreszierenden Zustand überführbar ist, und wobei eine räumlich hochaufgelöste Abbildung der Struktur erstellt wird,
indem die Probe mit einer vorgebbaren geringen Menge an Anregungslicht bestrahlt wird, so dass ein geringer Prozentsatz der Fluoreszenzmoleküle in den zweiten Zustand übergeht,
wobei das aus spontanem Zerfall der einzelnen angeregten Fluoreszenzmoleküle im zweiten Zustand resultierende Emissionslicht mittels einer Detektionseinrichtung nachgewiesen wird und für jedes dieser Fluoreszenzmoleküle mit Hilfe geeigneter statistischer Methoden der Schwerpunkt des Emissionslichts gebildet wird,
wobei die Fluoreszenzmoleküle im zweiten Zustand in den ersten Zustand zurückgeführt und/oder geblichen werden,
wobei die Schritte der Bestrahlung, der Detektion, der Schwerpunktbildung und der Rückführung in den ersten Zustand und/oder des Bleichens aller Moleküle im zweiten Zustand in einer hohen Anzahl für jeweils eine andere Untermenge an Fluoreszenzmolekülen wiederholt wird, und
wobei aus den so erstellten Einzelaufnahmen eine Gesamtabbildung erstellt wird.

Verfahren der hier in Rede stehenden Art sind seit einiger Zeit aus der Praxis bekannt. Lediglich beispielhaft seien an dieser Stelle die Mikroskopie-Verfahren STED (Stimulated Emission Depletion), RESOLFT (Reversible Saturable Optical Fluorescence Transitions), GSD (Ground State Depletion), Fluorescence Upconversion und PALM (photoactivated localization microscopy) genannt. Mit diesen abbildenden optischen Verfahren lassen sich räumliche Auflösungen jenseits der theoretischen Grenze erzielen, die gemäß dem Abbe'schen Gesetz durch die von der Wellenlänge des verwendeten Beleuchtungslichts abhängige Beugungsgrenze bestimmt wird.

Im Rahmen der STED-Mikroskopie wird eine Substanz in der zu untersuchenden Probe bereitgestellt, die durch Licht in einen angeregten Zustand versetzbar ist und aus diesem angeregten Zustand schlagartig abgeregt werden kann. Bei der STED-Mikroskopie werden als derartige Substanzen ganz überwiegend Fluoreszenzfarbstoffe eingesetzt. Im Allgemeinen wird die Substanz zunächst mit kurzwelligem Licht, bspw. durch einen grünen Laserpuls, in den angeregten Zustand überführt. Sodann wird die Substanz in einem Fokusrandbereich der Anregung mittels eines langwelligen (bspw. roten) Laserpulses gezielt abgeregt. Um eine Abregung der Substanz ausschließlich im Fokusrandbereich zu erreichen, wird die Abregungs-Punktfunktion speziell geformt. Hierzu werden im Allgemeinen Phasenfilter eingesetzt, die sich im Strahlengang des langwelligen Laserstrahls befinden und die Wellenfront des Abregungslichtstrahls ortsabhängig verändern. Entscheidend ist, dass der durch den Abregungslichtstrahl induzierte Übergang von dem angeregten in den abgeregten Zustand im Randbereich gesättigt, d.h. vollständig, stattfindet, so dass die Substanz lediglich in einem (prinzipiell beliebig) kleinen zentralen Bereich im angeregten Zustand verbleibt. Auf diese Weise wird mit dem Abregungslichtpuls eine Emission von Fluoreszenzlicht aus dem Randbereich des beugungsbegrenzten Anregungsflecks verhindert. Das detektierte Fluoreszenzlicht stammt somit aus einem eng definierten Probenbereich, dessen Durchmesser aufgrund der Sättigung der Abregung wesentlich kleiner sein kann als nach dem Abbe'schen Gesetz zulässig.

In der STED-Mikroskopie werden im Allgemeinen Farbstoffe als fluoreszierende Substanz zur Markierung der zu untersuchenden Struktur eingesetzt, die neben einer hohen Quantenausbeute den weiteren Vorteil besitzen, dass sie nicht so schnell ausbleichen wie normale Fluoreszenzproteine, bspw. das bekannte GFP (Green Fluorescence Protein). Die geringe Neigung zum Ausbleichen ist bei STED von ganz entscheidender Bedeutung, da die Probe dort zur gesättigten Abregung des fluoreszierenden Zustandes mit hohen Beleuchtungsintensitäten beaufschlagt werden muss.

In diesem Zusammenhang ist nachteilig, dass es sich bei den hier in Rede stehenden Fluoreszenzfarbstoffen um organische Moleküle handelt, denen geeignete chemische Gruppen fehlen, um an zu untersuchende Zielproteine zu binden. Dieser Umstand hat zur Folge, dass bei STED eine durch Immunfluoreszenz vermittelte Markierung eingesetzt wird. Dazu müssen die zu untersuchenden Zellen mit einem geeigneten Antikörper inkubiert werden, der das Zielprotein spezifisch erkennt. Problematisch ist insoweit, dass eine Markierung über Antikörper bei lebenden Zellen nicht möglich ist. Mit anderen Worten ist eine *in vivo* Markierung ausgeschlossen, so dass eine räumlich hochauflösende Untersuchung von innerhalb der lebenden Zelle ablaufenden physiologischen Reaktionen nicht möglich ist.

Der vorliegenden Erfindung liegt nunmehr die Aufgabe zugrunde, Verfahren der eingangs genannten Art zur räumlich hochauflösenden Untersuchung von einer mit einer fluoreszierenden Substanz markierten Struktur einer Probe derart auszugestalten und weiterzubilden, dass eine Anwendung auf zellbiologischer Ebene ermöglicht ist und physiologische Prozesse innerhalb lebender Zellen mit hoher Auflösung abbildbar sind.

Das erfindungsgemäße Verfahren löst die voranstehende Aufgabe einerseits durch die Merkmale des Patentanspruchs 1. Danach ist das insoweit gattungsbildende Verfahren derart ausgebildet, dass als Struktur, die mit der fluoreszierenden Substanz markiert wird, ein Protein - Zielprotein - in lebenden Zellen verwendet wird, indem ein die fluoreszierende Substanz umfassender Ligandkomplex über eine enzymatische Reaktion in der Zelle an ein Enzym gebunden wird, wobei das Enzym als Fusionsprotein gemeinsam mit dem zu untersuchenden Zielprotein exprimiert wird.

In erfindungsgemäßer Weise ist zunächst erkannt worden, dass eine hochauflösende Untersuchung von physiologischen, zellbiologischen und entwicklungsbiologischen Prozessen eine effiziente *in vivo* Markierung erfordert und dass eine Markierung über Antikörper demzufolge ausgeschlossen ist. Erfindungsgemäß wird ein Ligandkomplex bereitgestellt, der die fluoreszierende Substanz umfasst und den die Zelle über eine enzymatische Reaktion an ein Enzym bindet. Das Enzym wird gemeinsam mit dem Zielprotein als ein Fusionsprotein in der Zelle exprimiert, da die Gensequenz dieses Enzyms zuvor in das Genom der Zelle eingebracht wurde.

In vorteilhafter Weise ist vorgesehen, dass der Ligandkomplex einen reaktiven Linker aufweist, wobei der Ligandkomplex über den Linker kovalent an das Enzym gebunden wird. Die Zellen können dazu mit dem Ligandkomplex inkubiert werden und Ligandkomplexe, die nach einer vorgebbaren Inkubationszeit noch keine Bindung mit dem Enzym eingegangen sind, können ausgewaschen werden.

Im Rahmen einer besonders bevorzugten Ausführungsform wird als Enzym ein genetisch modifiziertes Hydrolase-Protein verwendet, bei dem die katalytische Base gegen einen Phenylalalin-Rest ersetzt ist. Da durch diesen Austausch die Fähigkeit des Enzyms, das Ester-Zwischenglied zu hydrolisieren ausgeschaltet wird, wird das Enzym katalytisch inaktiv, so dass eine stabile kovalente Bindung ausgebildet werden kann.

Im Hinblick auf eine hohe Vielfalt an Anwendungsmöglichkeiten kann vorgesehen sein, dass es sich bei der enzymatischen Reaktion, über die der Ligandkomplex an das Enzym bindet, um eine Halogenierung handelt. Die in diesem Fall zur Verfügung stehenden Ligandkomplexe, die einen reaktiven Linker mit einem entsprechenden Chloralkan aufweisen, können die Zellmembran durchdringen und wirken im Allgemeinen nicht toxisch auf die Zellen. Alternativ zu einer Halogenierungsreaktion sind auch eine Reduzierung oder eine Oxidierung als enzymatische Reaktionen denkbar.

Bei der fluoreszierenden Substanz, die neben dem reaktiven Linker als funktionaler Reporter den zweiten Baustein des Ligandkomplexes bildet, kann es sich um einen synthetischen Farbstoff handeln, wobei der Art des Farbstoffs prinzipiell keine Grenzen gesetzt sind. So kann es sich bei dem Fluoreszenzfarbstoff bspw. um TMR, einen Farbstoff mit hoher Quantenausbeute, hoher Photostabilität, geringer Triplett-Besetzung oder um einen funktionalen Reporter handeln, welche mit unterschiedlichen Anregungs- und Emissionswellenlängen zur Verfügung stehen.

So können bspw. Fluoreszenzfarbstoffe eingesetzt werden, bei denen es sich bei dem ersten und dem zweiten Zustand um mit unterschiedlichen Emissionswellenlängen fluoreszierende Zustände handelt. Alternativ ist es denkbar, dass es sich bei dem ersten Zustand um einen fluoreszierenden Zustand und bei dem zweiten Zustand um einen nicht fluoreszierenden Zustand handelt.

Zur Erstellung einer räumlich hochaufgelösten Abbildung der Zielproteine können als fluoreszierende Substanz auch schaltbare Fluoreszenzfarbstoffe verwendet werden, die durch Einstrahlen von Licht, durch chemische Reaktionen, durch Wärme oder auf andere Weise zwischen dem fluoreszierenden ersten Zustand und dem nicht fluoreszierenden zweiten Zustand umgeschaltet werden können. Dabei kann innerhalb der zu untersuchenden Probe bereichsweise zunächst der erste Zustand eingestellt werden. Sodann wird in einem Fokusrandbereich gesättigt der zweite Zustand eingeschaltet, so dass nur noch Fluoreszenzmoleküle in einem - beliebig - kleinen Fokusbereich im ersten Zustand verbleiben. Das von diesen Fluoreszenzmolekülen in Folge spontanen Zerfalls ausgehende Emissionslicht wird mittels einer Detektionseinrichtung nachgewiesen. Diese Methoden, bei denen die Beugungsgrenze durch einen reversiblen gesättigten optischen Übergang überwunden wird, werden allgemein als RESOLFT-Methoden bezeichnet. Einen Spezialfall stellt das STED-Verfahren dar, bei dem ein fluoreszierender Anregungszustand durch Einstrahlen von Licht mit einer geeigneten Abregungswellenlänge in einem Fokusrandbereich der Anregung gesättigt abgeregt wird.

Im Hinblick auf einen weiter verbesserten Informationsgewinn können im Rahmen der RESOLFT- bzw. STED-Verfahren zudem Pulse-Chase-Experimente für Mehrfarben-Applikationen durchgeführt werden. So können bspw. die Fusionsproteine bestehend aus Enzym und daran gebundenem Zielprotein zum Zeitpunkt t=0 mit "grünem" Farbstoff, zum Zeitpunkt t=1 mit "gelbem" und zum Zeitpunkt t=2 mit "rotem" Fluoreszenzfarbstoff markiert werden. Auf diese Weise ergibt sich bspw. die Möglichkeit, Membrantrafficking zeitlich und räumlich zu analysieren.

Die voranstehende Aufgabe ist des Weiteren durch ein Verfahren zur räumlich hochauflösenden Untersuchung von Proben gemäß Patentanspruch 12 gelöst.

Alternativ zu den beschriebenen RESOLFT-Verfahren bzw. dem STED-Verfahren wird bei dem erfindungsgemäßen Verfahren gemäß Patentanspruch 12 eine räumlich hochaufgelöste Abbildung der Zielproteine mittels eines statistischen Verfahrens erstellt. Dazu wird die Probe mit einer vorgebbaren geringen Menge an Anregungslicht bestrahlt, so dass nur ein geringer Prozentsatz der mit den Zielproteinen gekoppelten Fluoreszenzmoleküle in den zweiten, fluoreszierenden Zustand übergeht. In einem nächsten Schritt wird das aus spontanem Zerfall der einzelnen angeregten Fluoreszenzmoleküle im zweiten Zustand resultierende Emissionslicht mittels einer Detektionseinrichtung nachgewiesen. Die Menge des eingestrahlten Anregungslichts ist dabei so gewählt, dass das Detektionslicht der einzelnen angeregten Fluoreszenzmoleküle räumlich getrennt voneinander nachweisbar ist. Auf diese Weise wird die Möglichkeit geschaffen, für jedes angeregte Fluoreszenzmolekül mit Hilfe geeigneter statistischer Methoden den Schwerpunkt des Emissionslichts zu bilden. Die Schritte der Bestrahlung, der Detektion, der Schwerpunktbildung und der Rückführung in den ersten Zustand bzw. des Bleichens aller Moleküle im zweiten Zustand werden zeitlich hintereinander viele Male wiederholt, wobei in jedem Zyklus eine andere Untermenge an Fluoreszenzmolekülen angeregt wird. Der Vorgang kann bspw. in einer Größenordnung von 10.000 Mal wiederholt und aus den so jeweils erstellten Einzelaufnahmen eine Gesamtabbildung erstellt werden. Diese für sich gesehen bekannte Vorgehensweise wird im Allgemeinen als PALM (photoactivated localization microscopy) bezeichnet.

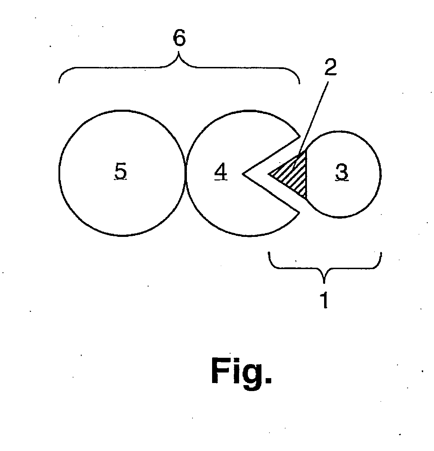

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigt die
- einzige Figur: in einer schematischen Darstellung ein Ausführungsbeispiel einer Bindung zwischen einem Zielprotein und einer fluoreszierenden Substanz, wie sie bei dem erfindungsgemäßen Verfahren zur räumlich hochauflösenden Untersuchung eingesetzt wird.

Die Fig. zeigt - schematisch - einen Ligandkomplex 1, der einen reaktiven Linker 2 sowie einen funktionalen Reporter in Form eines Fluoreszenzfarbstoffs 3 umfasst. Bei dem in der Fig. dargestellten Ausführungsbeispiel handelt es sich bei dem Fluoreszenzfarbstoff 3 konkret um einen "ATTO-Farbstoff", wie er von der Firma ATTO TEC bspw. unter der Produktbezeichnung ATTO 647 N für STED hergestellt wird.

Der Ligandkomplex 1 ist über eine enzymatische Halogenierung an ein Enzym 4 gebunden. Das Enzym 4 wird gemeinsam mit einem zu untersuchenden Zielprotein 5 als Fusionsprotein 6 exprimiert. Bei dem in dem Ausführungsbeispiel gemäß der Fig. dargestellten Enzym 4 handelt es sich konkret um ein von der Firma Promega unter dem Handelsnamen HaloTag^{™} vertriebenes Protein. Dieses Protein ist von einer prokaryotischen Hydrolase abgeleitet und kann verwendet werden, um eine N- oder C-terminale Fusion zu generieren, die in einer Vielzahl von Zellarten exprimiert werden kann.

Abschließend sei hervorgehoben, dass das voranstehend beschriebene Ausführungsbeispiel lediglich zur Erörterung der beanspruchten Lehre dient, diese jedoch nicht auf das Ausführungsbeispiel einschränkt.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

## Patentansprüche

1. Verfahren in der STED-Mikroskopie zur räumlich hochauflösenden Untersuchung von einer mit einer fluoreszierenden Substanz markierten Struktur einer Probe, wobei die Substanz wiederholt von einem ersten Zustand in einen zweiten Zustand überführbar ist, wobei sich die ersten und die zweiten Zustände in mindestens einer optischen Eigenschaft voneinander unterscheiden, umfassend die Schritte, dass die Substanz in einem zu erfassenden Probenbereich zunächst in den ersten Zustand gebracht wird und dass mittels eines optischen Signals der zweite Zustand induziert wird, wobei innerhalb des zu erfassenden Probenbereichs räumlich begrenzte Teilbereiche gezielt ausgespart werden, **dadurch gekennzeichnet, dass** als Struktur, die mit der fluoreszierenden Substanz markiert wird, ein Protein - Zielprotein (5) - in lebenden Zellen verwendet wird, indem ein die fluoreszierende Substanz umfassender Ligandkomplex (1) über eine enzymatische Reaktion in der Zelle an ein Enzym (4) gebunden wird, wobei das Enzym (4) als Fusionsprotein (6) gemeinsam mit dem zu untersuchenden Zielprotein (5) exprimiert wird.

2. Verfahren nach Anspruch 1, **dadurch gekennzeichnet, dass** der Ligandkomplex (1) über einen reaktiven Linker (2) kovalent an das Enzym (4) gebunden wird.

3. Verfahren nach Anspruch 1 oder 2, **dadurch gekennzeichnet, dass** als Enzym (4) ein genetisch modifiziertes Hydrolase-Protein verwendet wird, bei dem die katalytische Base gegen einen Phenylalanin-Rest ersetzt ist.

4. Verfahren nach einem der Ansprüche 1 bis 3, **dadurch gekennzeichnet, dass** es sich bei der enzymatischen Reaktion um eine Halogenierung handelt.

5. Verfahren nach einem der Ansprüche 1 bis 3, **dadurch gekennzeichnet, dass** es sich bei der enzymatischen Reaktion um eine Reduzierung oder um eine Oxidierung handelt.

6. Verfahren nach einem der Ansprüche 1 bis 5, **dadurch gekennzeichnet, dass** es sich bei der fluoreszierenden Substanz um einen synthetischen Fluoreszenzfarbstoff (3) handelt.

7. Verfahren nach Anspruch 6, **dadurch gekennzeichnet, dass** es sich bei dem Fluoreszenzfarbstoff (3) um einen Farbstoff mit hoher Quantenausbeute und/oder hoher Photostabilität und/oder geringer Triplett-Besetzung handelt.

8. Verfahren nach einem der Ansprüche 1 bis 7, **dadurch gekennzeichnet, dass** es sich bei dem ersten und dem zweiten Zustand um mit unterschiedlichen Emissionswellenlängen fluoreszierende Zustände handelt.

9. Verfahren nach einem der Ansprüche 1 bis 7, **dadurch gekennzeichnet, dass** es sich bei dem ersten Zustand um einen fluoreszierenden Zustand und bei dem zweiten Zustand um einen nicht fluoreszierenden Zustand handelt.

10. Verfahren nach Anspruch 9, **dadurch gekennzeichnet, dass** eine räumlich hochaufgelöste Abbildung der Zielproteine (5) erstellt wird,
indem die Probe zur bereichsweisen Erzeugung des fluoreszierenden ersten Zustandes mit Licht einer Wellenlänge des Anregungsspektrums der fluoreszierenden Substanz beleuchtet wird,
wobei die Probe zur gesättigten Erzeugung des nicht fluoreszierenden zweiten Zustandes in einem Fokusrandbereich der Anregung mit Licht einer geeigneten Abregungswellenlänge beleuchtet wird, und
wobei von der Probe ausgehendes Emissionslicht, das aus spontanem Zerfall verbliebener Moleküle im ersten Zustand in einem räumlich eng begrenzten Teilbereich resultiert, mittels einer Detektionseinrichtung nachgewiesen wird.

11. Verfahren nach Anspruch 10, **dadurch gekennzeichnet, dass** zur Ermittlung der Zeitabhängigkeit von physiologischen Prozessen Pulse-Chase Experimente mit unterschiedlich farbig fluoreszierenden Substanzen durchgeführt werden.

12. Verfahren zur räumlich hochauflösenden PALM (photoactivated localization microscopy) - Untersuchung von einer mit einer fluoreszierenden Substanz markierten Struktur einer Probe, wobei die Substanz von einem ersten Zustand in einen zweiten fluoreszierenden Zustand überführbar ist, und wobei eine räumlich hochaufgelöste Abbildung der Struktur erstellt wird,
indem die Probe mit einer vorgebbaren geringen Menge an Anregungslicht bestrahlt wird, so dass ein geringer Prozentsatz der Fluoreszenzmoleküle in den zweiten Zustand übergeht,
wobei das aus spontanem Zerfall der einzelnen angeregten Fluoreszenzmoleküle im zweiten Zustand resultierende Emissionslicht mittels einer Detektionseinrichtung nachgewiesen wird und für jedes dieser Fluoreszenzmoleküle mit Hilfe geeigneter statistischer Methoden der Schwerpunkt des Emissionslichts gebildet wird,
wobei die Fluoreszenzmoleküle im zweiten Zustand in den ersten Zustand zurückgeführt und/oder geblichen werden,
wobei die Schritte der Bestrahlung, der Detektion, der Schwerpunktbildung und der Rückführung in den ersten Zustand und/oder des Bleichens aller Moleküle im zweiten Zustand in einer hohen Anzahl für jeweils eine andere Untermenge an Fluoreszenzmolekülen wiederholt wird, und
wobei aus den so erstellten Einzelaufnahmen eine Gesamtabbildung erstellt wird,
**dadurch gekennzeichnet, dass** als Struktur, die mit der fluoreszierenden Substanz markiert wird, ein Protein - Zielprotein (5) - in lebenden Zellen verwendet wird, indem ein die fluoreszierende Substanz umfassender Ligandkomplex (1) über eine enzymatische Reaktion in der Zelle an ein Enzym (4) gebunden wird, wobei das Enzym (4) als Fusionsprotein (6) gemeinsam mit dem zu untersuchenden Zielprotein (5) exprimiert wird.

## Claims

1. STED microscopy method for high spatial resolution examination of a fluorescent substance-labelled structure of a sample, wherein the substance can be transferred repeatedly from a first state to a second state, wherein the first and second states differ from one another in at least one optical property, comprising the steps of the substance in a sample area to be recorded being initially brought to the first state and of the second state being induced by means of an optical signal, wherein spatially confined subareas are specifically omitted within the sample area to be recorded, **characterized in that** the fluorescent substance-labelled structure used is a protein - target protein (5) - in living cells, by binding a ligand complex (1) comprising the fluorescent substance to an enzyme (4) via an enzymatic reaction in the cell, wherein the enzyme (4) is expressed as a fusion protein (6) together with the target protein (5) to be examined.

2. Method according to Claim 1, **characterized in that** the ligand complex (1) is bound covalently to the enzyme (4) via a reactive linker (2).

3. Method according to Claim 1 or 2, **characterized in that** the enzyme (4) used is a genetically modified hydrolase protein in which the catalytic base has been replaced with a phenylalanine residue.

4. Method according to any of Claims 1 to 3, **characterized in that** the enzymatic reaction is halogenation.

5. Method according to any of Claims 1 to 3, **characterized in that** the enzymatic reaction is reduction or oxidation.

6. Method according to any of Claims 1 to 5, **characterized in that** the fluorescent substance is a synthetic fluorescent dye (3).

7. Method according to Claim 6, **characterized in that** the fluorescent dye (3) is a dye with a high quantum yield and/or high photostability and/or low triplet occupation.

8. Method according to any of Claims 1 to 7, **characterized in that** the first and second states are states fluorescing at different emission wavelengths.

9. Method according to any of Claims 1 to 7, **characterized in that** the first state is a fluorescent state and the second state is a non-fluorescent state.

10. Method according to Claim 9, **characterized in that** a high spatial resolution image of the target proteins (5) is produced
by illuminating the sample with light of a wavelength from the excitation spectrum of the fluorescent substance for area-by-area generation of the fluorescent first state,
wherein the sample is illuminated with light of a suitable de-excitation wavelength for saturated generation of the non-fluorescent second state in a focal boundary area of the excitation, and
wherein emission light which is emanating from the sample and which results from spontaneous decay of remaining molecules in the first state in a spatially narrowly confined subarea is detected by means of a detection device.

11. Method according to Claim 10, **characterized in that** the time dependence of physiological processes is determined by carrying out pulse-chase experiments with differently coloured fluorescent substances.

12. Method for high spatial resolution PALM (photoactivated localization microscopy) examination of a fluorescent substance-labelled structure of a sample, wherein the substance can be transferred from a first state to a second fluorescent state, and wherein a high spatial resolution image of the structure is produced,
by illuminating the sample with a specifiable small amount of excitation light in such a way that a small percentage of the fluorescent molecules transitions to the second state,
wherein the emission light resulting from spontaneous decay of the individual excited fluorescent molecules in the second state is detected by means of a detection device and suitable statistical methods are used to form the centroid of the emission light for each of these fluorescent molecules,
wherein the fluorescent molecules in the second state are returned to the first state and/or bleached,
wherein the steps of illumination, detection, centroid formation, and of returning to the first state and/or bleaching all molecules in the second state are repeated a large number of times for, in each case, a different subset of fluorescent molecules, and
wherein the individual pictures thus produced are used to produce an overall image,
**characterized in that** the fluorescent substance-labelled structure used is a protein - target protein (5) - in living cells, by binding a ligand complex (1) comprising the fluorescent substance to an enzyme (4) via an enzymatic reaction in the cell, wherein the enzyme (4) is expressed as a fusion protein (6) together with the target protein (5) to be examined.

## Revendications

1. Procédé, en microscopie STED, pour l'examen à haute résolution spatiale d'une structure d'un échantillon, marquée par une substance fluorescente, la substance pouvant d'une manière répétitive être convertie d'un premier état à un deuxième état, le premier et le deuxième états différant l'un de l'autre par au moins une propriété optique, comprenant les étapes consistant à mettre d'abord dans un premier état la substance se trouvant dans une zone de l'échantillon à détecter, et, à l'aide d'un signal optique, à induire le deuxième état, des zones de l'échantillon spatialement délimitées à l'intérieur de la zone de l'échantillon à détecter subissant un évidement ciblé, **caractérisé en ce qu'**on utilise en tant que structure marquée par la substance fluorescente une protéine - la protéine cible (5) - dans des cellules vivantes, par liaison à une enzyme (4) d'un complexe de ligands (1), comprenant la substance fluorescente, par l'intermédiaire d'une réaction enzymatique dans la cellule, l'enzyme (4) étant exprimée sous forme d'une protéine de fusion (6) en même temps que la protéine cible (5) à étudier.

2. Procédé selon la revendication 1, **caractérisé en ce que** le complexe de ligands (1) est lié d'une manière covalente à l'enzyme (4) par l'intermédiaire d'un lieur réactif (2).

3. Procédé selon la revendication 1 ou 2, **caractérisé en ce qu'**on utilise en tant qu'enzyme (4) une protéine hydrolase génétiquement modifiée, dans laquelle la base catalytique est remplacée par un résidu de phénylalanine.

4. Procédé selon l'une des revendications 1 à 3, **caractérisé en ce que**, pour ce qui concerne la réaction enzymatique, il s'agit d'une halogénation.

5. Procédé selon l'une des revendications 1 à 3, **caractérisé en ce que**, pour ce qui concerne la réaction enzymatique, il s'agit d'une réduction ou d'une oxydation.

6. Procédé selon l'une des revendications 1 à 5, **caractérisé en ce que**, pour ce qui concerne la substance fluorescente, il s'agit d'un colorant fluorescent synthétique (3).

7. Procédé selon la revendication 6, **caractérisé en ce que**, pour ce qui concerne le colorant fluorescent (3), il s'agit d'un colorant ayant un rendement quantique élevé et/ou une photostabilité élevée et/ou une faible occupation des triplets.

8. Procédé selon l'une des revendications 1 à 7, **caractérisé en ce que**, pour ce qui concerne le premier et le deuxième états, il s'agit d'états fluorescents ayant des longueurs d'onde d'émission différentes.

9. Procédé selon l'une des revendications 1 à 7, **caractérisé en ce que**, pour ce qui concerne le premier état, il s'agit d'un état fluorescent et, pour ce qui concerne le deuxième état, il s'agit d'un état non fluorescent.

10. Procédé selon la revendication 9, **caractérisé en ce qu'**une représentation à grande résolution spatiale des protéines cibles (5) est réalisée,
par le fait que l'on éclaire l'échantillon, pour la production, par zones, du premier état fluorescent à l'aide d'une lumière ayant une longueur d'onde du spectre d'excitation de la substance fluorescente, l'échantillon, pour une production saturée du deuxième état non fluorescent, est éclairé dans une zone marginale du foyer de l'excitation par une lumière ayant une longueur d'onde d'excitation appropriée, et on détecte à l'aide d'un dispositif de détection la lumière d'émission émanant de l'échantillon, lumière qui résulte d'une décomposition spontanée de molécules restantes dans un premier état, dans une zone partielle qui spatialement est étroitement délimitée.

11. Procédé selon la revendication 10, **caractérisé en ce que**, pour déterminer la dépendance des processus physiologiques vis-à-vis du temps, on procède à des expériences de marquage-localisation (Pulse-Chase) avec des substances présentant des fluorescences de couleurs différentes.

12. Procédé d'examen PALM (microscopie par localisation photoactivée) à haute résolution spatiale, d'une structure d'un échantillon marquée par une substance fluorescente, la substance pouvant être convertie d'un premier état à un deuxième état fluorescent, avec établissement d'une représentation à haute résolution spatiale de la structure, l'échantillon étant exposé à une quantité faible, pouvant être prédéfinie, de lumière d'excitation, de façon qu'un petit pourcentage des molécules fluorescentes passent au deuxième état,
la lumière d'émission résultant de la décomposition spontanée, dans le deuxième état, des molécules fluorescentes individuelles excitées, étant détectée à l'aide d'un dispositif de détection, et, pour chacune de ces molécules fluorescentes, le centre de gravité de la lumière d'émission étant formé à l'aide de méthodes statistiques appropriées,
les molécules fluorescentes se trouvant dans le deuxième état étant ramenées au premier état, et/ou blanchies,
des étapes d'irradiation, de détection, de formation du centre de gravité et de retour au premier état et/ou de blanchiment de l'ensemble de molécules se trouvant dans le deuxième état, étant répétées, en grand nombre, pour chaque sous-ensemble différent de molécules fluorescentes,
et
une représentation globale étant établie à partir des enregistrements individuels ainsi réalisés,
**caractérisé en ce qu'**on utilise en tant que structure marquée par la substance fluorescente une protéine - protéine cible (5) - dans des cellules vivantes, par liaison à une enzyme (4) d'un complexe de ligands (1), comprenant la substance fluorescente, par l'intermédiaire d'une réaction enzymatique dans la cellule, l'enzyme (4) étant exprimée en tant que protéine de fusion (6) en même temps que la protéine cible à étudier (5).
